(19) Europäisches Patentamt
European Patent Office
Office européen des brevets

(11) **EP 4 353 326 A1**

(12) **EUROPEAN PATENT APPLICATION**
published in accordance with Art. 153(4) EPC

(43) Date of publication:
**17.04.2024 Bulletin 2024/16**

(21) Application number: **22820203.2**

(22) Date of filing: **07.06.2022**

(51) International Patent Classification (IPC):
*A61Q 1/14* (2006.01)   *A61Q 19/10* (2006.01)
*A61K 8/02* (2006.01)   *A61K 8/37* (2006.01)
*A61K 8/39* (2006.01)   *A61K 8/86* (2006.01)

(52) Cooperative Patent Classification (CPC):
**A61K 8/02; A61K 8/33; A61K 8/34; A61K 8/37;
A61K 8/39; A61K 8/86; A61Q 1/14; A61Q 19/10**

(86) International application number:
**PCT/JP2022/022879**

(87) International publication number:
**WO 2022/260024 (15.12.2022 Gazette 2022/50)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **08.06.2021 JP 2021095896**

(71) Applicant: **Kao Corporation
Chuo-ku
Tokyo 103-8210 (JP)**

(72) Inventors:
• **TSUDA, Hiroko
Tokyo 131-8501 (JP)**
• **TAJIMA, Hitoshi
Tokyo 131-8501 (JP)**
• **OTSUKI, Yuta
Tokyo 131-8501 (JP)**

(74) Representative: **Hoffmann Eitle
Patent- und Rechtsanwälte PartmbB
Arabellastraße 30
81925 München (DE)**

(54) **AQUEOUS CLEANSER COMPOSITION**

(57)    Provided is an aqueous cleansing composition containing the following components (A), (B), (C), and (D): (A) a nonionic surfactant with a HLB of 13 or more, selected from the group consisting of (a1), (a2), and (a3): (a1) a polyoxyethylene lauric acid ester; (a2) a polyoxyethylene glyceryl (caprylate/caprate); and (a3) a polyoxyethylene glyceryl cocoate; (B) a nonionic surfactant with a HLB of 10 or less, selected from the group consisting of (b1), (b2), and (b3): (b1) a monofatty acid ester or ether of glycerin or diglycerin; (b2) a POE monofatty acid ester or POE glycerin monofatty acid ester having an average number of added moles of POE groups of from 1 to 5; and (b3) a sorbitan monofatty acid ester; (C) a polyol; and (D) water, wherein a mass ratio of the component (A) to the component (B), (A)/(B), is from 1 to 25.

EP 4 353 326 A1

**Description**

Field of the Invention

The present invention relates to an aqueous cleansing composition.

Background of the Invention

In recent years, for long lasting type base makeup cosmetics, a technology has been widely used in which oil-soluble silicone resins such as trimethylsiloxysilicate (TMS) are blended to form a hard, water-repellent makeup coating film on the skin surface not only to provide sweat sebum resistance, but also prevent makeup firm from smudging by physical friction. Since silicone resins used in this manner are oil-soluble, makeup cosmetics in which a large amount of these resins are blended are difficult to remove with aqueous cleansing agents, and need to be removed with makeup removers which use oil as the cleansing ingredients.

However, oil type and liquid type makeup removers which use oil as the cleansing ingredients are slimy, take a long time to remove along with dirt, and are sticky in the process of removal. Furthermore, they often leave sliminess and stickiness even after removal. For this reason, some people would then double cleanse their face with a facial cleanser, which resulted in prolonged skin friction for makeup removal.

On the other hand, more and more people are becoming aware of the importance of taking care of their skin and do not want to rub their skin with a makeup remover for a long time. As a result, it is getting to be difficult to achieve proper makeup removal without skin friction.

Aqueous cleansing cosmetics which can remove fat/oil dirt such as makeup are being considered.

For example, Patent Literature 1 describes that an aqueous cleansing cosmetic can remove fat/oil dirt such as makeup, suppresses stickiness and rough skin caused by excessive oil removal, and imparts persistent moist feeling, the aqueous cleansing cosmetic comprising a polyoxyethylene glyceryl (caprylate/caprate), a polyoxyethylene glyceryl cocoate, and a polyhydric alcohol.

(Patent Literature 1) JP-A-2010-70521

Summary of the Invention

The present invention relates to an aqueous cleansing composition containing the following components (A), (B), (C), and (D):

(A) a nonionic surfactant with a HLB of 13 or more, selected from the group consisting of (a1), (a2), and (a3) :

(a1) a polyoxyethylene laurate;
(a2) a polyoxyethylene glyceryl (caprylate/caprate); and
(a3) a polyoxyethylene glyceryl cocoate;

(B) a nonionic surfactant with a HLB of 10 or less, selected from the group consisting of (b1), (b2), and (b3) :

(b1) a monofatty acid ester or ether of glycerin or diglycerin;
(b2) a POE monofatty acid ester or POE glycerin monofatty acid ester having an average number of added moles of POE groups of from 1 to 5; and
(b3) a sorbitan monofatty acid ester;

(C) a polyol; and
(D) water,

wherein a mass ratio of the component (A) to the component (B), (A)/(B), is from 1 to 25.

Detailed Description of the Invention

Conventional aqueous cleansing cosmetics have difficulty in properly removing long lasting type base makeup cosmetics in which a large amount of oil-soluble silicone resins are blended.

The present invention relates to an aqueous cleansing composition which has high cleansing ability even for makeup cosmetics in which oil-soluble silicone resins such as TMS are blended to enhance long lasting effect.

The present inventors found that, by using a specific nonionic surfactant with a HLB of 13 or more, a specific nonionic surfactant with a HLB of 10 or less, and a polyol in combination in a specific ratio, an aqueous cleansing composition can be obtained which has high cleansing ability even for makeup cosmetics which contain oil-soluble silicone resins and the like to enhance long lasting effect.

The aqueous cleansing composition of the present invention can have high removal potential even for makeup cosmetics which form a hard coating film due to oil-soluble silicone resins such as TMS.

The component (A) is a nonionic surfactant with a HLB of 13 or more, selected from the group consisting of (a1), (a2), and (a3):

> (a1) a polyoxyethylene laurate;
> (a2) a polyoxyethylene glyceryl (caprylate/caprate); and
> (a3) a polyoxyethylene glyceryl cocoate.

The HLB is an index indicating the balance between hydrophilicity and lipophilicity (Hydrophile Lipophile Balance), and as used herein, it is determined by the following equation by Griffin.

$$HLB = 20 \times \frac{\text{Molecular weight of hydrophilic group moiety}}{\text{Molecular weight of surfactant}}$$

The component (A) is a hydrophilic nonionic surfactant with a specific structure, and works together with the component (B) to remove makeup cosmetics and the like with enhanced persistence. Therefore, it is preferable for the component (A) to have a certain degree of cleansing and removal potential on its own, and when combined with the component (B), a lipophilic nonionic surfactant, a clear improvement in removal potential can be seen.

The removal potential refers to, for example, the removal performance when makeup cosmetics applied onto a model substrate of pseudo-skin in Examples are removed by applying a predetermined physical force under certain physical conditions. It is ranked and determined by the removal rate calculated from color difference measurement of makeup cosmetics on the model substrate before and after cleansing.

The clear improvement in removal potential refers to, for example, an improvement in the removal potential in Examples by one or more ranks over that of the component (A) alone, and to reaching the rank A or B (removal rate of 50% or more) as an absolute level of removal potential.

From the point of ensuring water solubility, the nonionic surfactant of the component (A) has a HLB of 13 or more, and preferably 14 or more.

The component (A) preferably has an average number of added moles of polyoxyethylene (POE) groups of 9 moles or more for (a1), 6 moles or more for (a2), and 7 moles or more for (a3) from the viewpoint of having a HLB of 13 or more, preferably 30 moles or less for (a1), 20 moles or less for (a2), and 40 moles or less for (a3) from the viewpoint of makeup removal potential exerted by the component (A) alone, and more preferably 20 moles or less for (a1), 10 moles or less for (a2), and 20 moles or less for (a3). Specifically, POE (12) monolaurate is preferred as (a1), POE (6) glyceryl (caprylate/caprate) glyceryl and POE (7) glyceryl (caprylate/caprate) are preferred as (a2), and POE (7) glyceryl cocoate and POE (8) glyceryl cocoate are preferred as (a3).

One component (A) may be used, or two or more may be used in combination. The HLB in the case where two or more nonionic surfactants are used can be calculated by additive averaging the HLB values of the respective nonionic surfactant species based on their content ratio, which is referred to as the mixed HLB. Even when the HLB of any of (a1), (a2), and (a3) is less than 13, if their mixed HLB is 13 or more, they fall under the component (A) in the present invention.

The content of the component (A) is, from the viewpoint of cleansing ability (removal potential), ease of removal from the skin, and bare skin feeling after removal, preferably 3% by mass or more, more preferably 5% by mass or more, and further more preferably 10% by mass or more, and preferably 30% by mass or less, more preferably 25% by mass or less, and further more preferably 20% by mass or less, in the entire composition. The content of the component (A) is preferably from 3 to 30% by mass, more preferably from 5 to 25% by mass, and further more preferably from 10 to 20% by mass, in the entire composition.

The component (B) is a nonionic surfactant with a HLB of 10 or less, selected from the group consisting of (b1), (b2), and (b3) :

> (b1) a monofatty acid ester or ether of glycerin or diglycerin;
> (b2) a POE monofatty acid ester or POE glycerin monofatty acid ester having an average number of added moles of POE groups of from 1 to 5; and

(b3) a sorbitan monofatty acid ester.

The component (B) is a lipophilic nonionic surfactant with a specific structure, acts on the makeup coating film (coating film of foundation and the like) formed by oil-soluble silicone resins such as TMS to loosen its hard surface, and when combined with the component (A), is dissolved in water to provide high cleansing ability.

From the viewpoint of, in combination with the component (A), being actively adsorbed from the aqueous solution to the surface of the coating film to soften the coating film components, the nonionic surfactant of the component (B) has a HLB of 10 or less, and preferably 8 or less. From the viewpoint that an appropriate amount of the component (B) can be stably solubilized by the component (A), it preferably has a HLB of 2 or more, and more preferably 4 or more.

As for the component (B), from the viewpoint of having a HLB of 10 or less, examples of (b1) include a monoester of oleic acid, stearic acid, or isostearic acid and glycerin or diglycerin, and a monoether of oleyl alcohol, stearyl alcohol, or isostearyl alcohol and glycerin or diglycerin; examples of (b2) include a POE monomyristic acid ester, a POE monolauric acid ester, a POE glycerin monooleic acid ester, a POE glycerin monostearic acid ester, and a POE glycerin monoisostearic acid ester, having an average number of added moles of POE groups of from 1 to 5; and furthermore, examples of (b3) include sorbitan monostearate, sorbitan monooleate, and sorbitan monoisostearate.

Among the above, from the point of ensuring low-temperature stability, a monoester of oleic acid or isostearic acid and glycerin or diglycerin, or a monoether of oleyl alcohol or isostearyl alcohol and glycerin or diglycerin is suitably used as (b1); a POE monolauric acid ester, a POE glycerin monooleic acid ester, or a POE glycerin monoisostearic acid ester, having an average number of added moles of POE groups of from 1 to 5, is suitably used as (b2); and furthermore, sorbitan monooleate or sorbitan monoisostearate is suitably used as (b3).

One component (B) may be used, or two or more may be used in combination. Even when the HLB of any of (b1), (b2), and (b3) is greater than 10, if their mixed HLB is 10 or less, they fall under the component (B) in the present invention.

The content of the component (B) is, from the viewpoint of being solubilized by the component (A) and softening the surface of the coating film, preferably 0.3% by mass or more, more preferably 0.5% by mass or more, and further more preferably 1% by mass or more, and preferably 10% by mass or less, more preferably 8% by mass or less, and further more preferably 5% by mass or less, in the entire composition. The content of the component (B) is preferably from 0.3 to 10% by mass, more preferably from 0.5 to 8% by mass, and further more preferably from 1 to 5% by mass, in the entire composition.

In the present invention, the mass ratio of the component (A) to the component (B), (A)/(B), is important in order to increase the makeup removal potential. From the viewpoint of solubilizing the component (B), which has low solubility in water, with the component (A) to improve the removal potential, (A)/(B) is 1 or more, preferably 1.5 or more, and more preferably 2 or more. In addition, from the viewpoint of relatively increasing the component (B) sufficiently to improve the removal potential, (A)/(B) is 25 or less, preferably 15 or less, and more preferably 10 or less.

The mass ratio of the component (A) to the component (B), (A)/(B), is from 1 to 25, preferably from 1.5 to 15, and more preferably from 2 to 10.

Since the aqueous cleansing composition of the present invention is an aqueous solution of nonionic surfactants, it does not give the skin squeaky feeling after rinsing off like facial cleansers, but as it has the character that spontaneously mix in water, physical promote emulsification like oil doesn't need, and thus can be rinsed off quickly. From the viewpoint of reducing sliminess while rinsing from the skin keeping the makeup removal potential and providing bare skin feeling after removal, the total content of the components (A) and (B), ((A) + (B)), is preferably 5% by mass or more, and more preferably 7% by mass or more, in the entire composition. Also, from the viewpoint of reducing residual feeling on the skin after removal and effectively obtaining bare skin feeling after removal, it is preferably 25% by mass or less, and more preferably 20% by mass or less.

The total content of the components (A) and (B), ((A) + (B)), is preferably from 5 to 25% by mass, and more preferably from 7 to 20% by mass, in the entire composition.

The polyol of the component (C) promotes dissolution of the nonionic surfactants in water and facilitates their action on the makeup. Examples of such a polyol include those used in ordinary cleansing compositions, such as ethylene glycol, diethylene glycol, hexylene glycol, polyethylene glycol with an average molecular weight of 1 000 or less, propylene glycol, dipropylene glycol, polypropylene glycol with an average molecular weight of 1 500 or less, 1,2-propanediol, isoprene glycol, 1,3-butylene glycol, dibutylene glycol, glycerin, diglycerin, triglycerin, tetraglycerin, hexaglycerin, decaglycerin, and trimethylpropanol.

Among these, those generally raising the cloud point of POE addition type nonionic surfactants, such as 1,3-butylene glycol, dibutylene glycol, and 1,2-propanediol, are suitably used from the point of increasing solubility and expanding the temperature range in which transparency and uniformity are kept.

One polyol of the component (C) may be used, or two or more may be used in combination, and the content thereof is, from the viewpoint of promoting dissolution of the components (A) and (B) and allowing the cleansing ability to be exerted, preferably 5% by mass or more, more preferably 10% by mass or more, and further more preferably 20% by mass or more, and preferably 50% by mass or less, more preferably 45% by mass or less, and further more preferably 40% by mass or less, in the entire composition. The content of the component (C) is preferably from 5 to 50% by mass, more preferably from 10 to 45% by mass, and further more preferably from 20 to 40% by mass, in the entire composition.

In the present invention, water of the component (D) functions as a solvent, and the content thereof is, from the viewpoint of controlling the solubility of the surfactants together with the component (C) to allow the cleansing ability to be exerted, preferably 30% by mass or more, more preferably 40% by mass or more, and further more preferably 50% by mass or more, and preferably 80% by mass or less, more preferably 70% by mass or less, and further more preferably 60% by mass or less, in the entire composition. The content of water of the component (D) is preferably from 30 to 80% by mass, more preferably from 40 to 70% by mass, and further more preferably from 50 to 60% by mass, in the entire composition.

As described above, although the polyol of the component (C) has the role of extracting the removal potential for makeup while controlling the solubility of the components (A) and (B), a too small amount may not allow the cleansing ability of the surfactants to be fully exerted, whereas a too large amount may adversely affect the cleansing ability. Accordingly, the balance between polyol of the component (C) and water of the component (D) is important, and the mass ratio of the component (C) to the component (D), (C)/(D), is preferably 0.1 or more, and more preferably 0.25 or more, from the viewpoint of fully extracting the removal potential. In addition, from the viewpoint of avoiding an infinite increase in the critical micelle concentration (CMC) and suppressing separation of the lipophilic nonionic surfactant of the component (B) by breakage of the interaction with the hydrophilic nonionic surfactant of the component (A), it is preferably 1.3 or less, and more preferably 1.25 or less.

The mass ratio of the component (C) to the component (D), (C)/(D), is preferably from 0.1 to 1.3, and more preferably from 0.25 to 1.25.

In the present invention, the mass ratio of the total content of the components (A) and (B) to the component (D), ((A) + (B))/(D), is preferably 0.8 or less, more preferably 0.6 or less, and further more preferably 0.35 or less, from the viewpoint of removability such as rinsing ability and bare skin feeling after removal.

The aqueous cleansing composition of the present invention can further contain (E) one or more selected from the group consisting of an amphoteric surfactant and an anionic surfactant, in which case the removability such as rinsing ability and the bare skin feeling can be more enhanced. In addition, when filled into a foam dispensing container for use, the foam quality can be improved as well.

Examples of the amphoteric surfactant include those used in ordinary cleansing compositions, such as an alkylsulfobetaine, an alkylhydroxysulfobetaine, an alkylcarbobetaine, an alkylamidohydroxysulfobetaine, an alkylamidoamine type betaine, and an alkylimidazoline type betaine. The number of carbon atoms in the alkyl group of the amphoteric surfactant is preferably from 8 to 20, and more preferably from 8 to 14.

Among these, from the viewpoint of removability such as ease of rinsing and foam quality, the amphoteric surfactant is more preferably one or more selected from the group consisting of an alkylamidopropylbetaine and an alkylhydroxysulfobetaine, having an alkyl group having 8 to 14 carbon atoms, further more preferably an alkylhydroxysulfobetaine, and even more preferably laurylhydroxysulfobetaine.

One amphoteric surfactant may be used, or two or more may be used in combination, and the content thereof is, from the viewpoint of maintaining the cleansing ability while improving the bare skin feeling after cleansing, preferably 0.5% by mass or more, and more preferably 1% by mass or more, and from the viewpoint of maintaining the cleansing ability, preferably 4% by mass or less, and more preferably 3% by mass or less, in the entire composition.

The anionic surfactant is not restricted as long as it is any of those used in ordinary cleansing compositions, and examples thereof include a carboxylic acid surfactant such as a fatty acid salt, an alkyl ether carboxylic acid salt, and a polyoxyethylene alkyl ether carboxylic acid salt; a sulfuric acid ester such as an alkyl sulfuric acid salt, an alkyl ether sulfuric acid salt, and a polyoxyethylene alkyl ether sulfuric acid salt; a sulfonic acid salt such as a sulfosuccinic acid alkyl ester salt, a polyoxyalkylene sulfosuccinic acid alkyl ester salt, an $\alpha$-olefinsulfonic acid salt, and a hydroxyalkanesulfonic acid salt; and an N-acyl amino acid salt, an N-acyl alkyl taurine salt, and a POE phosphoric acid salt.

Among these, from the viewpoint of foamability and ensuring low-temperature stability, it is preferable for the anionic surfactant to contain at least one selected from the group consisting of a POE lauryl ether carboxylic acid salt, a POE lauryl ether sulfuric acid salt, and a POE phosphoric acid salt.

One anionic surfactant may be used, or two or more may be used in combination, and the content thereof is, from the viewpoint of maintaining the cleansing ability while improving the bare skin feeling, preferably 0.2% by mass or more, and more preferably 0.5% by mass or more, and from the viewpoint of maintaining the cleansing ability, preferably 2% by mass or less, and more preferably 1% by mass or less, in the entire composition.

One component (E) may be used, or two or more may be used in combination, and the amphoteric surfactant and the anionic surfactant can be used in combination.

The content of the component (E) is, from the viewpoint of maintaining the cleansing ability while improving the bare skin feeling, preferably 0.2% by mass or more, and more preferably 0.5% by mass or more, and preferably 4% by mass or less, and more preferably 3% by mass or less, in the entire composition. The content of the component (E) is, from the viewpoint of maintaining the cleansing ability, preferably from 0.2 to 4% by mass, and more preferably from 0.5 to 3% by mass, in the entire composition.

In the present invention, the mass ratio of the component (E) to the total content of the components (A) and (B), (E)/((A) + (B)), is preferably 0.03 or more, from the viewpoint of enhancing the removability such as rinsing ability and the bare skin feeling. On the other hand, from the viewpoint of avoiding the addition of a surfactant with a charge, changing the HLB of the system to hydrophilic, thereby reducing the efficiency of action on makeup, which is oily dirt, and affecting the makeup removal rate, (E)/((A) + (B)) is more preferably 0.15 or less.

The mass ratio of the component (E) to the total content of the components (A) and (B), (E)/((A) + (B)), is preferably from 0.03 to 0.15.

The aqueous cleansing composition of the present invention may further contain oily components (including a fragrance) other than the component (B) to the extent that the cleansing ability and transparency are not impaired. The content of the oily components (including a fragrance) other than the component (B) is, from the viewpoint of ease of removal from the skin, preferably 0.3% by mass or less, and more preferably 0.2% by mass or less, in the entire composition. From the same viewpoint, the content of components other than the fragrance among the oily components other than the component (B) is preferably 0.1% by mass or less, and more preferably 0.01% by mass or less, in the entire composition.

The aqueous cleansing composition of the present invention may further contain, in addition to the aforementioned components, components used in ordinary cleansing compositions. Examples thereof include a surfactant other than those described above; a low alcohol such as ethanol and isopropyl alcohol; an aromatic alcohol such as benzyl alcohol and benzyloxyethanol; a cellosolve such as ethyl cellosolve and butyl cellosolve; a carbitol such as ethyl carbitol and butyl carbitol; a moisturizing component such as a sugar (derivative), an amino acid (derivative), an animal or plant (protein) derivative, and an animal or plant extract; a silicone derivative such as a polyoxyalkylene modified silicone; a high molecular compound; an inorganic or organic salt such as sodium sulfate, sodium carbonate, sodium hydrogen carbonate, potassium chloride, sodium chloride, and sodium citrate; a pH adjuster such as an acid and an alkali; an antiinflammatory agent such as glycyrrhetinic acid, glycyrrhizic acid, and a derivative thereof; a bactericide such as isopropyl methylphenol; a preservative, a metal ion sequestering agent, an antioxidant, an ultraviolet absorber, a fragrance, a vitamin, a natural colorant, and a coloring agent such as a tar colorant.

The aqueous cleansing composition of the present invention is preferably liquid at 25°C. Being liquid refers to the property of immediately spreading out without shape retainability when released into the palm, and such a behavior is usually exhibited when the viscosity at 25°C is about 3 000 mPa·s or less. On the other hand, a lower viscosity is advantageous in that it spreads over the skin and blends with the makeup more easily, thereby shortening the time of scrubbing the skin. From this point, the viscosity at 25°C of the aqueous cleansing composition is preferably 100 mPa·s or less, more preferably 50 mPa·s or less, and further more preferably 30 mPa·s or less.

In the present invention, the viscosity of the aqueous cleansing composition is measured using a B-type viscometer (Model TVB-10 Viscometer, manufactured by Toki Sangyo Co., Ltd.) at 25°C for a measurement time of 1 min.

A viscosity of less than 100 mPa·s is measured at 60 rpm (number of rotations) of Rotor No. 1, a viscosity of from 100 to 199 mPa·s is measured at 30 rpm (number of rotations) of Rotor No. 1, a viscosity of from 200 to 499 mPa·s is measured at 12 rpm (number of rotations) of Rotor No. 1, and a viscosity of 1 000 mPa·s or more is measured at 12 rpm (number of rotations) of Rotor No. 2.

Since the aqueous cleansing composition of the present invention is water-based (aqueous), it easily mixes with water, can be easily rinsed off without sliminess, and provides enhanced bare skin feeling after rinsing off. Furthermore, when it is in liquid form (low viscosity) as described above, not only can these effects be improved, but it also easily spreads over the skin and the cleansing ability is quickly expressed. Also, a sheet or cosmetic cotton can be easily impregnated with the aqueous cleansing composition and used to wipe off dirt.

In particular, the viscosity at 25°C of the aqueous cleansing composition is preferably 30 mPa·s or less from the viewpoint of ease of impregnation into a sheet or cosmetic cotton in the case of being used to wipe off dirt, and excellent dispensability in the case of being filled into a foam dispensing container.

The aqueous cleansing composition of the present invention can be produced by pre-mixing (B) lipophilic nonionic surfactant with (A) hydrophilic nonionic surfactant, then adding (C) polyol and (D) water, and mixing them uniformly. The component (E) can be added as appropriate after the preparation of the aqueous solution.

The aqueous cleansing composition of the present invention is preferably a single transparent liquid phase at 25°C. Being a single transparent liquid phase means having a transparent appearance with no separation, and that each component is uniformly dissolved in each other. When in such a state, the interaction between the nonionic surfactants of the components (A) and (B) can increase the makeup removal potential, provide excellent removability from the skin, such as rinsing ability, and cleanses the skin providing it with high bare skin feeling. The appearance may become translucent when the surfactants form a higher-order association structure, but this case is also considered to be a single liquid phase. In the case where the system is separated into two phases, the appearance becomes cloudy or two-layered, and it becomes difficult to obtain the various performances described above as designed.

Since the aqueous cleansing composition of the present invention is an aqueous solution with the nonionic surfactants as the main cleaning ingredient, it is difficult to take it directly in hand and make it foam for use, but it is possible to force it into foam by, for example, placing it in a foam dispensing container and dispensing it out. By making the aqueous cleansing composition into foam, not only does it offer the convenience of homogeneous application to the skin while maintaining shape retainability, but it also offers the advantage of further improved makeup removability by applying it onto the skin as foam. In other words, the foam composed of the nonionic surfactant aqueous solution which is forced to foam although it does not originally foam immediately breaks all at once when encountering dirt. At that time, the interfacial energy possessed by the foam is converted into physical force, which acts on the surface of the makeup coating film. In particular, since the aqueous cleansing composition of the present invention has high removal potential, even the minute energy acting on the skin surface can be observed as a difference in makeup removability.

The aqueous cleansing composition of the present invention is preferably applied to the skin in a foam state from the point of ease of use and cleansing ability. From such a viewpoint, it is preferable that the aqueous cleansing composition is provided as a cleanser filled into an aerosol type or non-aerosol type foam dispensing container.

Although any foam dispensing container can be used as long as it is capable of mixing the cleansing composition with air and dispensing it as foam, a non-aerosol type foamer container is preferred from the point of convenience in an environment where the aqueous cleansing composition is used as a makeup remover and ease of disposal. Examples thereof include a pump foamer container which dispenses the aqueous cleansing composition by pushing the pump head, and a squeeze foamer container which dispenses the composition by pushing the body part. Specific examples thereof include foamer containers manufactured by Yoshino Kogyosho Co., Ltd. and Daiwa Can Company. In addition, for example, foamer containers equipped with the foaming mechanisms described in JP-A-7-315463, JP-A-8-230961, JP-A-2005-193972, JP-A-2019-107644, and the like can also be used. From the viewpoint of obtaining dense and elastic foam, a pump foamer container which dispenses the aqueous cleansing composition by pushing the pump head is preferred.

In particular, it is preferable to use a pump foamer container equipped with the gas-liquid mixing mechanism described in JP-A-2019-107644 since it has higher foaming performance and wider tolerance for the viscosity and properties of the solution. That is, as described in claim 1 of that publication, it is preferable that the gas-liquid mixing mechanism includes a liquid channel through which a liquid supplied to a mixing section from a liquid supply section passes and a gas channel through which a gas supplied to the mixing section from a gas supply section passes, the liquid channel including an adjacent liquid channel having a liquid inlet open to the mixing section, the gas channel including a plurality of adjacent gas channels each having a gas inlet open to the mixing section, the liquid inlet being located at a position corresponding to a confluent section of gases supplied to the mixing section via the gas inlet from the plurality of adjacent gas channels.

In pump foamer containers, the foam quality and pressing pressure upon application are easily affected by the viscosity of the contents. In order to ensure that the contents can be dispensed as foam in the daily temperature range and that the pressing pressure for dispensing can be applied without difficulty, the viscosity of the content, aqueous cleansing composition, is preferably 50 mPa·s or less at 25°C; however, in the case where the foamability of the solution itself is low, as in the present invention, the viscosity is more preferably 30 mPa·s or less.

The aqueous cleansing composition of the present invention is preferably filled into a foam dispensing container and provided as a foam type, and in other words, it is preferably used for a cleanser that includes the aqueous cleansing composition and a foam dispensing container containing the same.

The aqueous cleansing composition of the present invention is also suitable for use by impregnating a sheet or cosmetic cotton, applying it to the skin, and wiping off makeup and the like. After applying the aqueous cleansing composition to the skin, the aqueous cleansing composition may be removed by wiping it off together with makeup and the like using a sheet or cosmetic cotton.

The aqueous cleansing composition of the present invention is preferably used for skin cleansing, and in particular, is preferably used as a facial cleanser or makeup remover.

With respect to the embodiments mentioned above, the present invention further discloses the following compositions, cleansers, or cleansing methods.

<1> An aqueous cleansing composition comprising the following components (A), (B), (C), and (D):

(A) a nonionic surfactant with a HLB of 13 or more, selected from the group consisting of (a1), (a2), and (a3) :

(a1) a polyoxyethylene laurate;
(a2) a polyoxyethylene glyceryl (caprylate/caprate); and
(a3) a polyoxyethylene glyceryl cocoate;

(B) a nonionic surfactant with a HLB of 10 or less, selected from the group consisting of (b1), (b2), and (b3) :

(b1) a monofatty acid ester or ether of glycerin or diglycerin;

(b2) a POE monofatty acid ester or POE glycerin monofatty acid ester having an average number of added moles of POE groups of from 1 to 5; and

(b3) a sorbitan monofatty acid ester;

(C) a polyol; and

(D) water,

wherein a mass ratio of the component (A) to the component (B), (A)/(B), is from 1 to 25.

<2> The aqueous cleansing composition according to the above <1>, wherein the component (A) preferably has a HLB of 14 or more.

<3> The aqueous cleansing composition according to the above <1> or <2>, wherein the component (A) preferably has an average number of added moles of polyoxyethylene (POE) groups of 9 moles or more for (a1), 6 moles or more for (a2), and 7 moles or more for (a3).

<4> The aqueous cleansing composition according to any one of the above <1> to <3>, wherein the component (A) preferably has an average number of added moles of polyoxyethylene (POE) groups of 30 moles or less for (a1), 20 moles or less for (a2), and 40 moles or less for (a3), and more preferably 20 moles or less for (a1), 10 moles or less for (a2), and 20 moles or less for (a3).

<5> The aqueous cleansing composition according to any one of the above <1> to <4>, wherein, in the component (A), (a1) preferably contains POE (12) monolaurate, (a2) preferably contains one or two selected from the group consisting of POE (6) glyceryl caprylate/caprate and POE (7) glyceryl caprylate/caprate, and (a3) preferably contains one or two selected from the group consisting of POE (7) glyceryl cocoate and POE (8) glyceryl cocoate.

<6> The aqueous cleansing composition according to any one of the above <1> to <5>, wherein a content of the component (A) is preferably 3% by mass or more, more preferably 5% by mass or more, and further more preferably 10% by mass or more, in an entire composition.

<7> The aqueous cleansing composition according to any one of the above <1> to <6>, wherein a content of the component (A) is preferably 30% by mass or less, more preferably 25% by mass or less, and further more preferably 20% by mass or less, in an entire composition.

<8> The aqueous cleansing composition according to any one of the above <1> to <7>, wherein a content of the component (A) is preferably from 3 to 30% by mass, more preferably from 5 to 25% by mass, and further more preferably from 10 to 20% by mass, in an entire composition.

<9> The aqueous cleansing composition according to any one of the above <1> to <8>, wherein the component (B) preferably has a HLB of 8 or less.

<10> The aqueous cleansing composition according to any one of the above <1> to <9>, wherein the component (B) preferably has a HLB of 2 or more, and more preferably 4 or more.

<11> The aqueous cleansing composition according to any one of the above <1> to <10>, wherein, in the component (B), (b1) preferably contains one or more selected from the group consisting of a monoester of oleic acid, stearic acid, or isostearic acid and glycerin or diglycerin, and a monoether of oleyl alcohol, stearyl alcohol, or isostearyl alcohol and glycerin or diglycerin, and more preferably contains one or more selected from the group consisting of a monoester of oleic acid or isostearic acid and glycerin or diglycerin, and a monoether of oleyl alcohol or isostearyl alcohol and glycerin or diglycerin; (b2) preferably contains one or more selected from the group consisting of a POE monomyristic acid ester, a POE monolauric acid ester, a POE glycerin monooleic acid ester, a POE glycerin monostearic acid ester, and a POE glycerin monoisostearic acid ester, having an average number of added moles of POE groups of from 1 to 5, and more preferably contains one or more selected from the group consisting of a POE monolauric acid ester, a POE glycerin monooleic acid ester, and a POE glycerin monoisostearic acid ester, having an average number of added moles of POE groups of from 1 to 5; and (b3) preferably contains one or more selected from the group consisting of sorbitan monostearate, sorbitan monooleate, and sorbitan monoisostearate, and more preferably contains one or two selected from the group consisting of sorbitan monooleate and sorbitan monoisostearate.

<12> The aqueous cleansing composition according to any one of the above <1> to <11>, wherein a content of the component (B) is preferably 0.3% by mass or more, more preferably 0.5% by mass or more, and further more preferably 1% by mass or more, in an entire composition.

<13> The aqueous cleansing composition according to any one of the above <1> to <12>, wherein a content of the component (B) is preferably 10% by mass or less, more preferably 8% by mass or less, and further more preferably 5% by mass or less, in an entire composition.

<14> The aqueous cleansing composition according to any one of the above <1> to <13>, wherein a content of the component (B) is preferably from 0.3 to 10% by mass, more preferably from 0.5 to 8% by mass, and further more preferably from 1 to 5% by mass, in an entire composition.

<15> The aqueous cleansing composition according to any one of the above <1> to <14>, wherein a mass ratio of the component (A) to the component (B), (A)/(B), is preferably 1.5 or more, and more preferably 2 or more.

<16> The aqueous cleansing composition according to any one of the above <1> to <15>, wherein a mass ratio of the component (A) to the component (B), (A)/(B), is preferably 15 or less, and more preferably 10 or less.

<17> The aqueous cleansing composition according to any one of the above <1> to <16>, wherein a mass ratio of the component (A) to the component (B), (A)/(B), is preferably from 1.5 to 15, and more preferably from 2 to 10.

<18> The aqueous cleansing composition according to any one of the above <1> to <17>, wherein a total content of the components (A) and (B), ((A) + (B)), is preferably 5% by mass or more, and more preferably 7% by mass or more, in an entire composition.

<19> The aqueous cleansing composition according to any one of the above <1> to <18>, wherein a total content of the components (A) and (B), ((A) + (B)), is preferably 25% by mass or less, and more preferably 20% by mass or less, in an entire composition.

<20> The aqueous cleansing composition according to any one of the above <1> to <19>, wherein a total content of the components (A) and (B), ((A) + (B)), is preferably from 5 to 25% by mass, and more preferably from 7 to 20% by mass, in an entire composition.

<21> The aqueous cleansing composition according to any one of the above <1> to <20>, wherein the polyol of the component (C) preferably contains one or more selected from the group consisting of ethylene glycol, diethylene glycol, hexylene glycol, polyethylene glycol, propylene glycol, dipropylene glycol, polypropylene glycol, 1,2-propanediol, isoprene glycol, 1,3-butylene glycol, dibutylene glycol, glycerin, diglycerin, triglycerin, tetraglycerin, hexaglycerin, decaglycerin, and trimethylpropanol, and more preferably contains one or more selected from the group consisting of 1,3-butylene glycol, dibutylene glycol, and 1,2-propanediol.

<22> The aqueous cleansing composition according to any one of the above <1> to <21>, wherein a content of the component (C) is preferably 5% by mass or more, more preferably 10% by mass or more, and further more preferably 20% by mass or more, in an entire composition.

<23> The aqueous cleansing composition according to any one of the above <1> to <22>, wherein a content of the component (C) is preferably 50% by mass or less, more preferably 45% by mass or less, and further more preferably 40% by mass or less, in an entire composition.

<24> The aqueous cleansing composition according to any one of the above <1> to <23>, wherein a content of the component (C) is preferably from 5 to 50% by mass, more preferably from 10 to 45% by mass, and further more preferably from 20 to 40% by mass, in an entire composition.

<25> The aqueous cleansing composition according to any one of the above <1> to <24>, wherein a content of the component (D) is preferably 30% by mass or more, more preferably 40% by mass or more, and further more preferably 50% by mass or more, in an entire composition.

<26> The aqueous cleansing composition according to any one of the above <1> to <25>, wherein a content of the component (D) is preferably 80% by mass or less, more preferably 70% by mass or less, and further more preferably 60% by mass or less, in an entire composition.

<27> The aqueous cleansing composition according to any one of the above <1> to <26>, wherein a content of the component (D) is preferably from 30 to 80% by mass, more preferably from 40 to 70% by mass, and further more preferably from 50 to 60% by mass, in an entire composition.

<28> The aqueous cleansing composition according to any one of the above <1> to <27>, wherein a mass ratio of the component (C) to the component (D), (C)/(D), is preferably 0.1 or more, and more preferably 0.25 or more.

<29> The aqueous cleansing composition according to any one of the above <1> to <28>, wherein a mass ratio of the component (C) to the component (D), (C)/(D), is preferably 1.3 or less, and more preferably 1.25 or less.

<30> The aqueous cleansing composition according to any one of the above <1> to <29>, wherein a mass ratio of the component (C) to the component (D), (C)/(D), is preferably from 0.1 to 1.3, and more preferably from 0.25 to 1.25.

<31> The aqueous cleansing composition according to any one of the above <1> to <30>, preferably, further comprising (E) one or more selected from the group consisting of an amphoteric surfactant and an anionic surfactant.

<32> The aqueous cleansing composition according to the above <31>, wherein the amphoteric surfactant preferably contains one or more selected from the group consisting of an alkylsulfobetaine, an alkylhydroxysulfobetaine, an alkylcarbobetaine, an alkylamidohydroxysulfobetaine, an alkylamidoamine type betaine, and an alkylimidazoline type betaine.

<33> The aqueous cleansing composition according to the above <31> or <32>, wherein a number of carbon atoms in the alkyl group of the amphoteric surfactant is preferably from 8 to 20, and more preferably from 8 to 14 .

<34> The aqueous cleansing composition according to any one of the above <31> to <33>, wherein the amphoteric surfactant preferably contains one or more selected from the group consisting of an alkylamidopropylbetaine and an alkylhydroxysulfobetaine, having an alkyl group having 8 to 14 carbon atoms, more preferably contains an alkylhydroxysulfobetaine, and further more preferably contains laurylhydroxysulfobetaine.

<35> The aqueous cleansing composition according to any one of the above <31> to <34>, wherein a content of the amphoteric surfactant is preferably 0.5% by mass or more, and more preferably 1% by mass or more, in an entire composition.

<36> The aqueous cleansing composition according to any one of the above <31> to <35>, wherein a content of the amphoteric surfactant is preferably 4% by mass or less, and more preferably 3% by mass or less, in an entire composition.

<37> The aqueous cleansing composition according to the above <31>, wherein the anionic surfactant preferably contains one or more selected from the group consisting of a fatty acid salt, a carboxylic acid salt, a sulfuric acid salt, a sulfonic acid salt, a N-acyl amino acid salt, and a N-acyl alkyl taurine salt.

<38> The aqueous cleansing composition according to the above <31> or <37>, wherein the anionic surfactant is preferably a carboxylic acid salt, and more preferably contains one or two selected from the group consisting of an alkyl ether carboxylic acid salt and a polyoxyethylene alkyl ether carboxylic acid salt.

<39> The aqueous cleansing composition according to the above <31> or <37>, wherein the anionic surfactant is preferably a sulfuric acid salt, and more preferably contains one or two selected from the group consisting of an alkyl sulfuric acid salt, an alkyl ether sulfuric acid salt, and a polyoxyethylene alkyl ether sulfuric acid salt.

<40> The aqueous cleansing composition according to the above <31> or <37>, wherein the anionic surfactant is preferably a sulfonic acid salt, and more preferably contains one or more selected from the group consisting of a sulfosuccinic acid alkyl ester salt, a polyoxyalkylene sulfosuccinic acid alkyl ester salt, an $\alpha$-olefinsulfonic acid salt, and a hydroxyalkanesulfonic acid salt.

<41> The aqueous cleansing composition according to the above <31>, wherein the anionic surfactant preferably contains one or more selected from the group consisting of a POE lauryl ether carboxylic acid salt, a POE lauryl ether sulfuric acid salt, and a POE phosphoric acid salt.

<42> The aqueous cleansing composition according to any one of the above <31>, <37> to <41>, wherein a content of the anionic surfactant is preferably 0.2% by mass or more, and more preferably 0.5% by mass or more, in an entire composition.

<43> The aqueous cleansing composition according to any one of the above <31>, <37> to <42>, wherein a content of the anionic surfactant is preferably 2% by mass or less, and more preferably 1% by mass or less, in an entire composition.

<44> The aqueous cleansing composition according to any one of the above <31> to <43>, wherein a content of the component (E) is preferably 0.2% by mass or more, and more preferably 0.5% by mass or more, in an entire composition.

<45> The aqueous cleansing composition according to any one of the above <31> to <44>, wherein a content of the component (E) is preferably 4% by mass or less, and more preferably 3% by mass or less, in an entire composition.

<46> The aqueous cleansing composition according to any one of the above <31> to <45>, wherein a content of the component (E) is preferably from 0.2 to 4% by mass, and more preferably from 0.5 to 3% by mass, in an entire composition.

<47> The aqueous cleansing composition according to any one of the above <31> to <46>, wherein a mass ratio of the component (E) to a total content of the components (A) and (B), (E)/((A) + (B)), is preferably 0.03 or more.

<48> The aqueous cleansing composition according to any one of the above <31> to <47>, wherein a mass ratio of the component (E) to a total content of the components (A) and (B), (E)/((A) + (B)), is preferably 0.15 or less.

<49> The aqueous cleansing composition according to any one of the above <31> to <48>, wherein a mass ratio of the component (E) to a total content of the components (A) and (B), (E)/((A) + (B)), is preferably from 0.03 to 0.15.

<50> The aqueous cleansing composition according to any one of the above <1> to <49>, preferably, further comprising oily components (including a fragrance) other than the component (B).

<51> The aqueous cleansing composition according to the above <50>, wherein a content of the oily components (including a fragrance) other than the component (B) is preferably 0.3% by mass or less, and more preferably 0.2% by mass or less, in an entire composition.

<52> The aqueous cleansing composition according to the above <50>, wherein a content of oily components other than the fragrance among the oily components other than the component (B) is preferably 0.1% by mass or less, and more preferably 0.01% by mass or less, in an entire composition.

<53> The aqueous cleansing composition according to any one of the above <1> to <52>, which is liquid at 25°C.

<54> The aqueous cleansing composition according to any one of the above <1> to <53>, which has a viscosity at 25°C of preferably 3 000 mPa·s or less, more preferably 100 mPa·s or less, further more preferably 50 mPa·s or less, and even more preferably 30 mPa·s or less.

<55> The aqueous cleansing composition according to any one of the above <1> to <54>, which is a single transparent liquid phase at 25°C.

<56> The aqueous cleansing composition according to any one of the above <1> to <55>, which is contained in a foam dispensing container for use.

<57> The aqueous cleansing composition according to any one of the above <1> to <56>, which is filled into an aerosol type or non-aerosol type foamer container for use.

<58> The aqueous cleansing composition according to the above <57>, wherein the non-aerosol type foamer container is preferably a pump foamer container or squeeze foamer container.

<59> The aqueous cleansing composition according to the above <58>, wherein the pump foamer container is a container described in claim 1 of JP-A-2019-107644.

<60> The aqueous cleansing composition according to any one of the above <1> to <59>, which is for skin cleansing, and is preferably used as a facial cleanser or makeup remover.

<61> A cleanser comprising a foam dispensing container and the aqueous cleansing composition according to any one of the above <1> to <60>, contained in the foam dispensing container.

<62> A cleanser comprising a sheet or cosmetic cotton, and the aqueous cleansing composition according to any one of the above <1> to <60>, with which the sheet or the cosmetic cotton is impregnated.

<63> A method for cleansing skin in which the aqueous cleansing composition according to any one of the above <1> to <60> is contained in a foam dispensing container, dispensed as foam, and applied to the skin.

<64> A method for cleansing skin in which a sheet or cosmetic cotton is impregnated with the aqueous cleansing composition according to any one of the above <1> to <60>, and the sheet or the cosmetic cotton is applied to the skin to wipe off dirt.

Examples

Examples 1 to 9 and Comparative Examples 1 to 15

Aqueous cleansing compositions with the compositional features shown in Table 1 and Table 2 were produced by pre-mixing (B) lipophilic nonionic surfactant with (A) hydrophilic nonionic surfactant, then adding (C) polyol and (D) water, and mixing them uniformly. For the obtained aqueous cleansing compositions, the removal potential and solution state were evaluated. The results are shown together in Table 1.

Note that, in the table of Examples, the content of each component indicates the active amount.

(Evaluation methods)

(1) Removal potential:

To a 5 cm × 10 cm range on a white artificial leather sheet (manufactured by Okamoto Kaseihin Co., Ltd., Laforet White) cut to 7 cm × 12 cm, foundation (Revlon ColorStay Makeup N, color No. 370) was applied uniformly with fingers to reach from 0.04 to 0.05 g, then allowed to dry for 2 hours or longer. Thereafter, the artificial leather sheet was placed on an electronic balance, 2 drops (from 0.04 to 0.05 g) of each aqueous cleansing composition were applied, and while applying a force of 50 g weight with one finger, 10 round trips were made slowly over the same location as if drawing a line (about 1 sec for 5 cm each way), and then the sheet was rinsed with running water.

Using a color difference meter, the respective color differences (L, a, b) of the white artificial leather sheet before foundation application, after foundation application, and after cleansing were measured in three locations, the cleansing removal rate was determined by calculation, and the average value was determined, which was then evaluated in accordance with the following criteria.

A: removal rate of 60% or more.
B: removal rate of from 50 to 59%.
C: removal rate of from 40 to 49%.
D: removal rate of from 10 to 39%.
E: removal rate of 9% or less (no potential).

(2) Solution state:

Each aqueous cleansing composition was placed in a screw tube and visually observed for appearance, which is indicated in accordance with the following criteria.

T: transparent.
ST: semitransparent.
C: cloudy white or separation into two layers.

[Table 1]

| | | | HLB | Example | | | Comparative Example | | | | |
|---|---|---|---|---|---|---|---|---|---|---|---|
| | | | | 1 | 2 | 3 | 1 | 2 | 3 | 4 | 5 |
| A-a1 | POE (12) monolaurate | EMANON 1112 (Kao Corporation) | 14 | 13 | | | 15 | | | | |
| A-a2 | POE (6) glyceryl caprylate/ caprate | TEGOSOFT GMC-6 (EVONIC industries AG) | 15 | | 13 | | | 15 | | | |
| A-a3 | POE (7) glyceryl cocoate | LEVENOL C301 (Kao Corporation) | 13 | | | 13 | | | 15 | | |
| Other components | POE (20) sorbitan laurate | RHEODOL TW-L120 (Kao Corporation) | 15 | | | | | | | 13 | |
| | Polyglyceryl-10 laurate | SUNSOFT M12-JW (50%) (Taiyo Kagaku Co., Ltd.) | 15 | | | | | | | | 13 |
| B | Diglycerin isostearate | COSMOL 41 (The Nisshin OilliO Group, Ltd.) | 5 | 2 | 2 | 2 | | | | 2 | 2 |
| C | 1,3-Butylene glycol | 1,3 Butylene glycol-P (KH Neochem Co., Ltd.) | | 30 | 30 | 30 | 30 | 30 | 30 | 30 | 30 |
| D | Water | | | 55 | 55 | 55 | 55 | 55 | 55 | 55 | 55 |
| | Total | | | 100 | 100 | 100 | 100 | 100 | 100 | 100 | 100 |
| | A/B | | | 6.5 | 6.5 | 6.5 | - | - | - | - | - |
| | Removal potential | | | B | A | B | D | C | D | E | E |
| | Solution state | | | T | T | T | T | T | T | T | C |

[Table 2]

| | | | HLB | Example | | | | | | Comparative Example | | | | | | | | | |
|---|---|---|---|---|---|---|---|---|---|---|---|---|---|---|---|---|---|---|---|
| | | | | 4 | 5 | 6 | 7 | 8 | 9 | 6 | 7 | 8 | 9 | 10 | 11 | 12 | 13 | 14 | 15 |
| A-a1 | POE (12) monolaurate | EMANON 1112 (Kao Corporation) | 14 | 13 | 13 | 13 | 13 | 13 | 13 | 15 | 13 | 13 | 13 | 13 | 13 | 13 | 13 | 13 | 13 |
| B-b1 | Isostearyl glyceryl ether | PENETOL GE-IS (Kao Corporation) | 3 | 2 | | | | | | | | | | | | | | | |
| B-b1 | Glyceryl monoisostearate | GWIS-100 (Nihon Emulsion Co., Ltd.) | 3 | | 2 | | | | | | | | | | | | | | |
| B-b1 | Diglycerin isostearate | COSMOL 41 (The Nisshin OilliO Group, Ltd.) | 5 | | | 2 | | | | | | | | | | | | | |
| B-b3 | Sorbitan monoisostearate | NIKKOL SI10-R (Nikko Chemicals Co., Ltd.) | 5 | | | | 2 | | | | | | | | | | | | |
| B-b2 | POE (3) monoisostearate | PEIS03 (Nihon Emulsion Co., Ltd.) | 7 | | | | | 2 | | | | | | | | | | | |
| B-b1 | 2-Ethylhexyl glyceryl ether | PENETOL GE-EH (Kao Corporation) | 10 | | | | | | 2 | | | | | | | | | | |

| | | | | Example | | | | | | Comparative Example | | | | | | | | | |
|---|---|---|---|---|---|---|---|---|---|---|---|---|---|---|---|---|---|---|---|
| | | | HLB | 4 | 5 | 6 | 7 | 8 | 9 | 6 | 7 | 8 | 9 | 10 | 11 | 12 | 13 | 14 | 15 |
| Other components | POE (5) hydrogenated castor oil | HCO5 (Nihon Emulsion Co., Ltd.) | 5 | | | | | | | | 2 | | | | | | | | |
| | POE (6) glyceryl monoisostearate | GWIS-106 (Nihon Emulsion Co., Ltd.) | 8 | | | | | | | | | 2 | | | | | | | |
| | POE (6) monoisostearate | PEIS06 (Nihon Emulsion Co., Ltd.) | 9 | | | | | | | | | | 2 | | | | | | |
| | POE (6) sorbitan monooleate | RHEODOL TW-0106 (Kao Corporation) | 10 | | | | | | | | | | | 2 | | | | | |
| | POE (10) glyceryl monoisostearate | GWIS-110 (Nihon Emulsion Co., Ltd.) | 10 | | | | | | | | | | | | 2 | | | | |
| | POE (20) glyceryl diisostearate | GWIS-220 (Nihon Emulsion Co., Ltd.) | 10 | | | | | | | | | | | | | 2 | | | |
| | POE (30) sorbit tetraoleate | RHEODOL 430V (Kao Corporation) | 11 | | | | | | | | | | | | | | 2 | | |
| | POE (60) hydrogenated castor oil | EMANON CH60 (Kao Corporation) | 14 | | | | | | | | | | | | | | | 2 | |
| | POE (20) hydrogenated castor oil | HC20 (Nihon Emulsion Co., Ltd.) | 14 | | | | | | | | | | | | | | | | 2 |

14

EP 4 353 326 A1

(continued)

| | | HLB | Example | | | | | | Comparative Example | | | | | | | | | | |
| | | | 4 | 5 | 6 | 7 | 8 | 9 | 6 | 7 | 8 | 9 | 10 | 11 | 12 | 13 | 14 | 15 |
| C | 1,3-Butylene glycol | 1,3 Butylene glycol-P (KH Neochem Co., Ltd.) | 30 | 30 | 30 | 30 | 30 | 30 | 30 | 30 | 30 | 30 | 30 | 30 | 30 | 30 | 30 | 30 |
| D | Water | | 55 | 55 | 55 | 55 | 55 | 55 | 55 | 55 | 55 | 55 | 55 | 55 | 55 | 55 | 55 | 55 |
| | Total | | 100 | 100 | 100 | 100 | 100 | 100 | 100 | 100 | 100 | 100 | 100 | 100 | 100 | 100 | 100 | 100 |
| | A/B | | 6.5 | 6.5 | 6.5 | 6.5 | 6.5 | 6.5 | - | - | - | - | - | - | - | - | - | - |
| | Removal potential | | A | A | A | A | B | B | D | A | C | C | C | D | D | C | D | D |
| | Solution state | | T | T | T | T | T | T | T | C | T | T | T | T | T | T | T | T |

Examples 10 to 28 and Comparative Examples 16 to 18

Aqueous cleansing compositions with the compositional features shown in Table 3 to Table 6 were produced by pre-mixing (B) lipophilic nonionic surfactant with (A) hydrophilic nonionic surfactant, then adding (C) polyol and (D) water, and mixing them uniformly. For the obtained aqueous cleansing compositions, the removal potential and solution state were evaluated in the same manner as in Examples 1 to 9. In addition, the ease of rinsing and the bare skin feeling were evaluated. The results are shown together in Table 3 to Table 6.

(Evaluation methods)

(3) Ease of rinsing:

1 g of each aqueous cleansing composition was applied and spread on a forearm, then rinsed off, and five expert panelists made a sensory evaluation on whether the composition could be rinsed off without sliminess. The results are indicated by the number of expert panelists who evaluated the composition as being able to be rinsed off without sliminess.

(4) Bare skin feeling:

1 g of each aqueous cleansing composition was applied and spread on a forearm, then rinsed off, and five expert panelists made a sensory evaluation on the bare skin feeling (no residual sliminess) of the skin immediately after the rinsing off. The results are indicated by the number of expert panelists who evaluated the composition as providing the skin with high bare skin feeling (no residual sliminess).

[Table 3]

| | | | | Example | | | | | Comparative Example | | |
|---|---|---|---|---|---|---|---|---|---|---|---|
| | | | HLB | 10 | 11 | 12 | 13 | 14 | 16 | 17 | 18 |
| A-a2 | POE (6) glyceryl caprylate/ caprate | TEGOSOFT GMC-6 (EVONIC industries AG) | 15 | 14.25 | 13.5 | 12 | 10.5 | 7.5 | 15 | 14.75 | 5 |
| B-b1 | Diglycerin isostearate | COSMOL 41 (The Nisshin OilliO Group, Ltd.) | 5 | 0.75 | 1.5 | 3 | 4.5 | 7.5 | | 0.25 | 10 |
| C | 1,3-Butylene glycol | 1,3 Butylene glycol-P (KH Neochem Co., Ltd.) | | 30 | 30 | 30 | 30 | 30 | 30 | 30 | 33 |
| D | Water | | | 55 | 55 | 55 | 55 | 55 | 55 | 55 | 55 |
| | Total | | | 100 | 100 | 100 | 100 | 100 | 100 | 100 | 103 |
| | A/B | | | 19 | 9 | 4 | 2.3 | 1 | - | 59 | 0.5 |
| | Removal potential | | | B | A | A | A | B | C | C | C |
| | Solution state | | | T | T | T | T | ST | T | T | C |
| | Ease of rinsing | | | 3 | 3 | 4 | 4 | 4 | 3 | 3 | 2 |
| | Bare skin feeling | | | 4 | 4 | 5 | 5 | 3 | 4 | 4 | 1 |

[Table 4]

|  |  |  | HLB | Example | | | |
|---|---|---|---|---|---|---|---|
|  |  |  |  | 15 | 16 | 17 | 18 |
| A-a2 | POE (6) glyceryl caprylate/caprate | TEGOSOFT GMC-6 (EVONIC industries AG) | 15 | 6 | 12 | 18 | 24 |
| B-b1 | Diglycerin isostearate | COSMOL 41 (The Nisshin OilliO Group, Ltd.) | 5 | 1.5 | 3 | 4.5 | 6 |
| C | 1,3-Butylene glycol | 1,3 Butylene glycol-P (KH Neochem Co., Ltd.) |  | 30 | 30 | 30 | 30 |
| D | Water |  |  | 62.5 | 55 | 47.5 | 40 |
|  | Total |  |  | 100 | 100 | 100 | 100 |
|  | A/B |  |  | 4 | 4 | 4 | 4 |
|  | (A+B)/D |  |  | 0.12 | 0.27 | 0.47 | 0.75 |
|  | Removal potential |  |  | B | A | A | A |
|  | Solution state |  |  | T | T | T | ST |
|  | Ease of rinsing |  |  | 4 | 4 | 4 | 3 |
|  | Bare skin feeling |  |  | 4 | 4 | 3 | 2 |

[Table 5]

|  |  |  | HLB | Example | | | |
|---|---|---|---|---|---|---|---|
|  |  |  |  | 19 | 20 | 21 | 22 |
| A-a2 | POE (6) glyceryl caprylate/caprate | TEGOSOFT GMC-6 (EVONIC industries AG) | 15 | 12 | 12 | 12 | 12 |
| B-b1 | Diglycerin isostearate | COSMOL 41 (The Nisshin OilliO Group, Ltd.) | 5 | 3 | 3 | 3 | 3 |
| C | Dipropylene glycol | DPG-RF (Adeka Corporation) |  | 10 | 15 | 30 | 45 |
| D | Water |  |  | 75 | 70 | 55 | 40 |
|  | Total |  |  | 100 | 100 | 100 | 100 |
|  | A/B |  |  | 4 | 4 | 4 | 4 |
|  | C/D |  |  | 0.13 | 0.21 | 0.55 | 1.125 |
|  | Removal potential |  |  | B | B | A | A |
|  | Solution state |  |  | T | T | T | T |
|  | Ease of rinsing |  |  | 3 | 3 | 4 | 4 |
|  | Bare skin feeling |  |  | 4 | 4 | 4 | 4 |

[Table 6]

| | Component name | | HL B | Example | | | | | |
|---|---|---|---|---|---|---|---|---|---|
| | | | | 23 | 24 | 25 | 26 | 27 | 28 |
| A-a1 | POE (12) monolaurate | EMANON 1112 (Kao Corporation) | 14 | 6 | 6 | 6 | 6 | 6 | 6 |
| A-a2 | POE (6) glyceryl caprylate/caprate | TEGOSOFT GMC-6 (EVONIC industries AG) | 15 | 6 | 6 | 6 | 6 | 6 | 6 |
| B-b1 | Diglycerin isostearate | COSMOL 41 (The Nisshin OilliO Group, Ltd.) | 5 | 3 | 3 | 3 | 3 | 3 | 3 |
| C | Dipropylene glycol | DPG-RF (Adeka Corporation) | | 4 | 4 | 4 | 4 | 4 | 4 |
| C | 1,3-Butylene glycol | 1,3 Butylene glycol-P (KH Neochem Co., Ltd.) | | 20 | 20 | 20 | 20 | 20 | 20 |
| C | Glycerin | Glycerin (cosmetic grade) (Kao Corporation) | | 6 | 6 | 6 | 6 | 6 | 6 |
| Other component s | PPG (9) diglyceryl ether | SY-DP9 (Sakamoto Yakuhin Kogyo Co., Ltd.) | | 3 | 3 | 3 | 3 | 3 | 3 |
| E | Laurylhydroxysulfobetaine | Amphitol 20HD (33%) (Kao Corporation) * formulation is act. | | | 1 | 2 | | | 1 |
| E | Sodium POE (2) lauryl ether sulfate | Emal 227 (27%) (Kao Corporation) * formulation is act. | | | | | 0.5 | 1 | 0.5 |
| D | Water | | | 52 | 51 | 50 | 51.5 | 51 | 50.5 |
| | Total | | | 100 | 100 | 100 | 100 | 100 | 100 |
| | A/B | | | 4 | 4 | 4 | 4 | 4 | 4 |
| | E/(A+B) | | | 0 | 0.067 | 0.133 | 0.033 | 0.067 | 0.1 |
| | Removal potential | | | A | A | B | A | A | B |
| | Solution state | | | T | T | T | T | T | T |
| | Ease of rinsing | | | 3 | 3 | 4 | 5 | 4 | 4 |
| | Bare skin feeling | | | 3 | 4 | 4 | 5 | 5 | 5 |

Examples 29 to 33

Aqueous cleansing compositions with the compositional features shown in Table 7 were produced by pre-mixing (B) lipophilic nonionic surfactant with (A) hydrophilic nonionic surfactant, then adding (C) polyol and (D) water, and mixing them uniformly. The component (E) was added after the preparation of the aqueous solution and mixed uniformly. For the obtained aqueous cleansing compositions, the removal potential and solution state were evaluated in the same manner as in Examples 1 to 9, and the ease of rinsing and bare skin feeling were evaluated in the same manner as in Examples 10 to 28. In addition, the foam quality and the removal rate reflecting actual use were evaluated. The results are shown together in Table 7.

(Evaluation methods)

(5) Foam quality:

Each aqueous cleansing composition was filled into foam dispensing containers (foam dispensing container A: manufactured by Yoshino Kogyosho Co., Ltd., general-purpose pump foamer, and foam dispensing container B: manufactured by Yoshino Kogyosho Co, Ltd., pump foamer equipped with the gas-liquid mixing mechanism described in JP-A-2019-107644), one push of foam was then dispensed into the palm, and the foam quality was evaluated in accordance with the following criteria.

A: homogeneous foam.
B: some larger foam is mixed in.
C: overall, foam is large and inhomogeneous.
D: no foam is formed.
E: unable to dispense the composition from pump.

(6) Removal rate reflecting actual use:

In order to measure the removal rate when the aqueous cleansing composition is applied as foam, it is necessary to make evaluation assuming an area where the composition, whose volume has been increased by turning it into foam, can act uniformly. Therefore, it was treated by a method similar to actual use, in which foundation applied to a large area was massaged with fingers, and the removal rate was measured.
To a 5 cm $\times$ 10 cm range on a white artificial leather sheet (manufactured by Okamoto Kaseihin Co., Ltd., Laforet White) cut to 7 cm $\times$ 12 cm, foundation (Revlon ColorStay Makeup N, color No. 370) was applied uniformly with fingers to reach from 0.04 to 0.05 g, then allowed to dry for 2 hours or longer.
Thereafter, 0.2 g of each aqueous cleansing composition was applied as liquid or turned into foam, placed on the sheet, and crushed, and then while applying a force of about 70 g weight with two fingers, the entire application surface was lightly massaged by drawing circles of about 3 cm in diameter overlapping each other 10 times to blend the foundation with the composition. After that, the aqueous cleansing composition was rinsed off with running water.
Note that, in the case of turning the aqueous cleansing composition into foam, it was filled into a foam dispensing container (dispensing container B) and foam was dispensed from that container.
Using a color difference meter, the respective color differences (L, a, b) of the white artificial leather sheet before foundation application, after foundation application, and after cleansing were measured in ten locations, the cleansing removal rate was determined by calculation, and the average value was determined, which was then evaluated in accordance with the following criteria.
SA: removal rate of 60% or more.

A: removal rate of from 50 to 59%.
B: removal rate of from 40 to 49%.
C: removal rate of from 30 to 39%.
D: removal rate of 29% or less.

[Table 7]

| | | | HLB | Example | | | | |
|---|---|---|---|---|---|---|---|---|
| | | | | 29 | 30 | 31 | 32 | 33 |
| A-a1 | POE (12) monolaurate | EMANON 1112 (Kao Corporation) | 14 | 6 | 6 | 6 | 6 | 6 |
| A-a2 | POE (6) glyceryl caprylate/ caprate | TEGOSOFT GMC-6 (EVONIC industries AG) | 15 | 6 | 6 | 6 | 6 | 6 |
| B-b1 | Diglycerin isostearate | COSMOL 41 (The Nisshin OilliO Group, Ltd.) | 5 | 0.75 | 3 | 3 | 3 | 3 |
| C | Dipropylene glycol | DPG-RF (Adeka Corporation) | | 4 | 4 | 4 | 4 | 4 |
| C | 1,3-Butylene glycol | 1,3 Butylene glycol-P (KH Neochem Co., Ltd.) | | 20 | 20 | 20 | 20 | 20 |
| C | Glycerin | Glycerin (cosmetic grade) (Kao Corporation) | | 6 | 6 | 6 | 6 | 6 |
| Other components | PPG (9) diglyceryl ether | SY-DP9 (Sakamoto Yakuhin Kogyo Co., Ltd.) | | 3 | 3 | 3 | 3 | 3 |
| E | Laurylhydroxysulfobetaine | Amphitol 20HD (33%) (Kao Corporation) * formulation is act. | | | | 1 | | 1 |
| E | Sodium POE (2) lauryl ether sulfate | Emal 227 (27%) (Kao Corporation) * formulation is act. | | | | | 1 | 0.5 |
| D | Water | | | 54.25 | 52 | 51 | 51 | 50.5 |
| | Total | | | 100 | 100 | 100 | 100 | 100 |
| | A/B | | | 16 | 4 | 4 | 4 | 4 |
| | Removal potential | | | A | A | A | A | B |
| | Solution state | | | T | T | T | T | T |
| | Ease of rinsing | | | 3 | 3 | 3 | 4 | 4 |
| | Bare skin feeling | | | 3 | 3 | 4 | 5 | 5 |
| | Foam quality (foam dispensing container A) | | | B | C | B | B | A |
| | Foam quality (foam dispensing container B) | | | A | A | A | A | A |
| | Removal rate by evaluation method reflecting actual use (applied as liquid) | | | B | A | A | B | B |
| | Removal rate by evaluation method reflecting actual use (applied as foam) | | | A | SA | SA | SA | A |

## EP 4 353 326 A1

Examples 34 to 38

Aqueous cleansing compositions with the compositional features shown in Table 8 were produced by premixing (B) lipophilic nonionic surfactant with (A) hydrophilic nonionic surfactant, then adding (C) polyol and (D) water, and mixing them uniformly. For the obtained aqueous cleansing compositions, the removal potential and solution state were evaluated in the same manner as in Examples 1 to 9, and the ease of rinsing and bare skin feeling were evaluated in the same manner as in Examples 10 to 28. In addition, the ease of impregnating a cosmetic cotton was evaluated, assuming wiping upon application. Furthermore, the foam quality was evaluated in the same manner as in Examples 29 to 33. The results are shown together in Table 8.

(Evaluation methods)

(7) Ease of impregnating cosmetic cotton:

1 mL of each aqueous cleansing composition was placed on a cosmetic cotton, and how it permeated was observed, which was evaluated in accordance with the following criteria.

A: immediately permeates.
B: the liquid pools on the surface for a moment and then promptly permeates.
C: the liquid pools on the surface for from 2 to 3 sec and then gradually permeates.
D: the liquid pools on the surface and does not permeate unless physical force such as bending is applied.
E: the liquid pools on the surface and does not permeate sufficiently even when physical force is applied.

[Table 8]

| | | | | | Example | | | | |
|---|---|---|---|---|---|---|---|---|---|
| | | | HLB | 34 | 35 | 36 | 37 | 38 |
| A-a2 | POE (6) glyceryl caprylate/caprate | TEGOSOFT GMC-6 (EVONIC industries AG) | 15 | 12 | 12 | 12 | 12 | 12 |
| B-b1 | Diglycerin isostearate | COSMOL 41 (The Nisshin OilliO Group, Ltd.) | 5 | 3 | 3 | 3 | 3 | 3 |
| Other components | Acrylic acid-alkyl methacrylate copolymer | Carbopol ETD (Lubrizol Advanced Materials, Inc.) | | | | | | 0.04 |
| | 48% KOH | 48% KOH (Occidental Chemical Corporation) | | | | | | 0.02 |
| | PEG6000 | K-PEG6000LA (Kao Corporation) | | | 3 | 2.2 | | |
| | PEG (150) monostearate | EMANON 3199V (Kao Corporation) | | | | 1.8 | 3 | |
| C | 1,3-Butylene glycol | 1,3 Butylene glycol-P (KH Neochem Co., Ltd.) | | 30 | 27 | 27 | 27 | 30 |
| D | Water | | | 55 | 55 | 54 | 55 | 54.94 |
| | Total | | | 100 | 100 | 100 | 100 | 100 |
| | A/B | | | 4 | 4 | 4 | 4 | 4 |
| | Viscosity (mPa·s) | | | 14 | 18 | 27 | 34 | 62 |
| | Removal potential | | | A | A | A | A | A |

(continued)

| | | | HLB | Example | | | | |
|---|---|---|---|---|---|---|---|---|
| | | | | 34 | 35 | 36 | 37 | 38 |
| Solution state | | | | T | T | T | T | ST |
| Ease of rinsing | | | | 4 | 4 | 3 | 3 | 3 |
| Bare skin feeling | | | | 5 | 4 | 4 | 4 | 4 |
| Ease of impregnating cosmetic cotton upon wiping | | | | A | B | C | C | C |
| Foam quality (foam dispensing container A) | | | | C | C | C | D | D |
| Foam quality (foam dispensing container B) | | | | A | A | A | B | C |

Example 39 and Comparative Example 19

Aqueous cleansing compositions with the compositional features shown in Table 9 were produced by pre-mixing (B) lipophilic nonionic surfactant with (A) hydrophilic nonionic surfactant, then adding (C) polyol and (D) water, and mixing them uniformly. For the obtained aqueous cleansing compositions, the solution state was evaluated in the same manner as in Examples 1 to 9. In addition, the removal potential when wiped off using a cotton, the lack of stickiness in the process of drying, and the lack of residual feeling on the skin after drying (that is, after use) were evaluated. The results are shown together in Table 9.

(Evaluation methods)

(8) Removal potential:

To a 5 cm × 10 cm range on a white artificial leather sheet (manufactured by Okamoto Kaseihin Co., Ltd., Laforet White) cut to 7 cm × 12 cm, foundation (Revlon ColorStay Makeup N, color No. 370) was applied uniformly with fingers to reach from 0.04 to 0.05 g, then allowed to dry for 2 hours or longer. A cosmetic cotton cut into a 1.5 cm square was impregnated with the aqueous cleansing composition to 800% by mass, placed on the foundation-applied side of the sheet placed on an electronic balance, and slow wiping was performed twice in a straight line while applying a force of 100 g weight with one finger.

Using a color difference meter, the respective color differences (L, a, b) of the white artificial leather sheet before foundation application, after foundation application, and after cleansing were measured in three locations, the cleansing removal rate was determined by calculation, and the average value was determined, which was then evaluated in accordance with the following criteria.

A: removal rate of 60% or more.
B: removal rate of from 50 to 59%.
C: removal rate of from 40 to 49%.
D: removal rate of from 10 to 39%.
E: removal rate of 9% or less (no potential).

(9) Lack of stickiness in the process of drying and lack of residual feeling on skin after drying:

Using a cosmetic cotton impregnated with 1.5 g of each aqueous cleansing composition, a forearm was wiped several times, and five expert panelists made sensory evaluations on the lack of stickiness in the process of skin drying (upon drying) and the lack of residual feeling after drying. The results are indicated by the number of expert panelists who evaluated the composition as providing less stickiness in the process of drying and less residual feeling on the skin.

[Table 9]

| | | | HLB | Example 39 | Comparative Example 19 |
|---|---|---|---|---|---|
| A-a2 | POE (6) glyceryl caprylate/caprate | TEGOSOFT GMC-6 (EVONIC industries AG) | 15 | 6 | 7.5 |
| B-b1 | Diglycerin isostearate | COSMOL 41 (The Nisshin OilliO Group, Ltd.) | 5 | 1.5 | |
| C | 1,3-Butylene glycol | 1,3 Butylene glycol-P (KH Neochem Co., Ltd.) | | 15 | 15 |
| D | Water | | | 77.5 | 77.5 |
| | Total | | | 100 | 100 |
| | A/B | | | 4 | - |
| | Removal potential | | | A | C |
| | Solution state | | | ST | T |
| | Lack of stickiness in the process of drying | | | 3 | 2 |
| | Lack of residual feeling on skin after drying | | | 4 | 3 |

Examples 40 to 41

Aqueous cleansing compositions with the compositional features shown in Table 10 were produced by pre-mixing (B) lipophilic nonionic surfactant and oil (isododecane) with (A) hydrophilic nonionic surfactant, then adding (C) polyol and (D) water, and mixing them uniformly. For the obtained aqueous cleansing compositions, the removal potential and solution state were evaluated in the same manner as in Examples 1 to 9. In addition, the ease of rinsing and the bare skin feeling were evaluated in the same manner as in Examples 10 to 28. The results are shown together in Table 10.

[Table 10]

| | | | | Example | |
|---|---|---|---|---|---|
| | | | HL B | 40 | 41 |
| A-a2 | POE (6) glyceryl caprylate/caprate | TEGOSOFT GMC-6 (EVONIC industries AG) | 14 | 13 | 13 |
| B-b1 | Diglycerin isostearate | COSMOL 41 (The Nisshin OilliO Group, Ltd.) | 5 | 2 | 2 |
| C | 1,3-Butylene glycol | 1,3 Butylene glycol-P (KH Neochem Co., Ltd.) | | 30 | 30 |
| | Isododecane | Marukasol R | | | 0.1 |
| D | Water | | | 55 | 54. 9 |
| | Total | | | 100 | 100 |
| | A/B | | | 6.5 | 6.5 |
| | Removal potential | | | A | A |
| | Solution state | | | T | T |
| | Ease of rinsing | | | 4 | 3 |
| | Bare skin feeling | | | 4 | 4 |

## Claims

1. An aqueous cleansing composition comprising the following components (A), (B), (C), and (D):

(A) a nonionic surfactant with a HLB of 13 or more, selected from the group consisting of (a1), (a2), and (a3) :

> (a1) a polyoxyethylene laurate;
> (a2) a polyoxyethylene glyceryl (caprylate/caprate); and
> (a3) a polyoxyethylene glyceryl cocoate;

(B) a nonionic surfactant with a HLB of 10 or less, selected from the group consisting of (b1), (b2), and (b3) :

> (b1) a monofatty acid ester or ether of glycerin or diglycerin;
> (b2) a POE monofatty acid ester or POE glycerin monofatty acid ester having an average number of added moles of POE groups of from 1 to 5; and
> (b3) a sorbitan monofatty acid ester;

(C) a polyol; and
(D) water,

wherein a mass ratio of the component (A) to the component (B), (A)/(B), is from 1 to 25.

2. The aqueous cleansing composition according to claim 1, which is contained in a foam dispensing container for use.

3. The aqueous cleansing composition according to claim 1 or 2, which is liquid at 25°C.

4. The aqueous cleansing composition according to any one of claims 1 to 3, which has a viscosity at 25°C of 100 mPa·s or less.

5. The aqueous cleansing composition according to any one of claims 1 to 4, which is a single transparent liquid phase at 25°C.

6. The aqueous cleansing composition according to any one of claims 1 to 5, wherein a total content of the components (A) and (B) is from 5 to 25% by mass.

7. The aqueous cleansing composition according to any one of claims 1 to 6, further comprising (E) one or more selected from the group consisting of an amphoteric surfactant and an anionic surfactant, wherein a mass ratio of the component (E) to a total content of the components (A) and (B), (E)/((A) + (B)), is from 0.03 to 0.25.

8. The aqueous cleansing composition according to any one of claims 1 to 7, wherein a content of the component (A) is from 3 to 30% by mass in an entire composition.

9. The aqueous cleansing composition according to any one of claims 1 to 8, wherein a content of the component (B) is from 0.3 to 10% by mass in an entire composition.

10. The aqueous cleansing composition according to any one of claims 1 to 9, wherein a content of the component (C) is from 5 to 50% by mass in an entire composition.

11. The aqueous cleansing composition according to any one of claims 1 to 10, wherein a content of the component (D) is from 30 to 80% by mass in an entire composition.

12. The aqueous cleansing composition according to any one of claims 1 to 11, wherein a mass ratio of the component (C) to the component (D), (C)/(D), is from 0.1 to 1.3.

13. The aqueous cleansing composition according to any one of claims 1 to 12, wherein a content of oily components (including a fragrance) other than the component (B) is 0.3% by mass or less in an entire composition.

14. The aqueous cleansing composition according to claim 13, wherein a content of components other than the fragrance among the oily components other than the component (B) is 0.1% by mass or less in an entire composition.

15. The aqueous cleansing composition according to any one of claims 1 to 14, which is for skin cleansing.

## INTERNATIONAL SEARCH REPORT

| International application No. |
| --- |
| **PCT/JP2022/022879** |

**A. CLASSIFICATION OF SUBJECT MATTER**

*A61Q 1/14*(2006.01)i; *A61Q 19/10*(2006.01)i; *A61K 8/02*(2006.01)i; *A61K 8/37*(2006.01)i; *A61K 8/39*(2006.01)i;
*A61K 8/86*(2006.01)i
FI:    A61K8/86; A61K8/39; A61K8/37; A61Q19/10; A61K8/02; A61Q1/14

According to International Patent Classification (IPC) or to both national classification and IPC

**B. FIELDS SEARCHED**

Minimum documentation searched (classification system followed by classification symbols)

A61Q1/14; A61Q19/10; A61K8/02; A61K8/37; A61K8/39; A61K8/86

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

Mintel GNPD

**C. DOCUMENTS CONSIDERED TO BE RELEVANT**

| Category* | Citation of document, with indication, where appropriate, of the relevant passages | Relevant to claim No. |
| --- | --- | --- |
| X | WO 2016/002713 A1 (KAO CORP.) 07 January 2016 (2016-01-07) claims, paragraphs [0057], [0066], [0186], table 3, example 13 | 1, 3-12, 15 |
| Y | | 2 |
| A | | 13-14 |
| Y | JP 2004-277365 A (POLA CHEMICAL INDUSTRIES, INC.) 07 October 2004 (2004-10-07) claims, paragraphs [0007]-[0009], [0011] | 2 |
| Y | JP 2011-213682 A (NARIS COSMETICS CO., LTD.) 27 October 2011 (2011-10-27) claims, paragraph [0031] | 2 |
| Y | JP 2018-193461 A (KOBAYASHI PHARMACEUTICAL CO., LTD.) 06 December 2018 (2018-12-06) claims, paragraph [0051] | 2 |
| A | JP 2013-112633 A (DKS CO., LTD.) 10 June 2013 (2013-06-10) entire text | 1-15 |

☐ Further documents are listed in the continuation of Box C.  ☑ See patent family annex.

| \* Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **27 July 2022** | **16 August 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
Information on patent family members

International application No.

**PCT/JP2022/022879**

| Patent document cited in search report | Publication date (day/month/year) | Patent family member(s) | Publication date (day/month/year) |
|---|---|---|---|
| WO 2016/002713 A1 | 07 January 2016 | US 2017/0151159 A1 claims, paragraphs [0096], [0114], [0116], table 3, example 13 CN 106659654 A | |
| JP 2004-277365 A | 07 October 2004 | (Family: none) | |
| JP 2011-213682 A | 27 October 2011 | (Family: none) | |
| JP 2018-193461 A | 06 December 2018 | (Family: none) | |
| JP 2013-112633 A | 10 June 2013 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 7315463 A
- JP 8230961 A
- JP 2005193972 A
- JP 2019107644 A